(19) Europäisches Patentamt / European Patent Office / Office européen des brevets

(11) EP 3 654 346 A1

(12) EUROPEAN PATENT APPLICATION

(43) Date of publication:
20.05.2020 Bulletin 2020/21

(51) Int Cl.:
G16H 40/63 (2018.01)        G06F 3/0346 (2013.01)
A61B 5/11 (2006.01)

(21) Application number: 18206636.5

(22) Date of filing: 16.11.2018

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Koninklijke Philips N.V.
5656 AG Eindhoven (NL)

(72) Inventors:
• AARTS, Vincent Alexander Rudolf
5656 AE Eindhoven (NL)
• RISPENS, Sietse Menno
5656 AE Eindhoven (NL)

(74) Representative: de Haan, Poul Erik et al
Philips International B.V.
Philips Intellectual Property & Standards
High Tech Campus 5
5656 AE Eindhoven (NL)

(54) **DETERMINING A TRANSFORMATION MATRIX**

(57) According to an aspect, there is provided a method of operating an apparatus to determine a transformation matrix for measurements from an accelerometer that is worn by a subject, the apparatus comprising a memory unit arranged to store information for several activity events, the method comprising (i) detecting (202) an activity event in acceleration measurements obtained by the accelerometer, wherein the subject is in one or more postures during the activity event; (ii) determining (204) at least one quality indicator related to the detected activity event; (iii) determining (206) whether each of the at least one quality indicators meets a respective predetermined criterion; (iv) responsive to at least one quality indicator meeting the respective predetermined criterion, deciding (208) whether information related to the detected activity event can be stored in the memory unit, and if so, storing information related to the detected activity event in the memory unit together with information related to one or more other activity events if any; (v) determining (210) whether a calibration procedure should be performed based on information stored in the memory unit, and if so, processing the stored information to determine a transformation matrix, wherein the transformation matrix relates a frame of reference of the accelerometer to a frame of reference of the subject.

Fig. 1

**Description**

FIELD OF THE INVENTION

This disclosure relates to the determination of a transformation matrix for measurements from an accelerometer that is worn by a subject.

BACKGROUND OF THE INVENTION

It is desirable to be able to monitor a health condition or an activity level of a subject. Such monitoring of the subject may include determining the posture of the subject, and for how long the subject has been in that posture. For example, if through monitoring of the subject, it is determined that the subject has been in the supine posture for an excessive period of time, then the subject may be at risk of developing decubitus ulcers. In another example, if through monitoring of the subject, it is determined that the posture of the subject has changed suddenly (for example, from the standing posture to lying prone) over a short period of time, then the subject may have suffered a fall. Information on the posture of the subject is also useful in determining the activity level of the subject or the activity that the subject is performing. For example, the subject will need to be in an upright posture when walking, and so walking detection can make use of information on the posture of the subject.

It is possible to monitor the posture of a subject using an accelerometer that may be worn by, or affixed to, the subject, for example as part of a wearable device. In order to correctly determine the posture and/or activity of the subject from these accelerometer measurements, the orientation of the accelerometer with respect to the body axes of the subject (also referred to herein as the 'frame of reference' of the subject) must be determined. Thus, it is necessary to perform a calibration procedure for the accelerometer in order to determine the orientation of accelerometer with respect to the body axes of the subject. The calibration procedure can determine a transformation matrix that relates the frame of reference of the accelerometer to the frame of reference/body axes of the subject.

An example of performing a calibration procedure on a wearable device is described in US 2013/116602. This document provides means for determining an orientation of a body-mounted or implanted device relative to the body, with the device having an orientation detection unit, and an uncontrolled output of the orientation detection unit over a period of time together with one or more reference conditions defined in a body reference system are used for determining the relative orientation of the device and hence for calibration.

It has been found that calibration methods based on a preliminary calibration can produce incorrect posture outputs over time as the preliminary calibration may be incorrect and/or inaccurate.

SUMMARY OF THE INVENTION

There is therefore a need for an improved method and apparatus for determining a transformation matrix that relates a frame of reference of the accelerometer to a frame of reference of the subject.

According to a first specific aspect, there is provided a method of operating an apparatus to determine a transformation matrix for measurements from an accelerometer that is worn by a subject. The apparatus comprises a memory unit arranged to store information for several activity events, and the method comprises (i) detecting an activity event in acceleration measurements obtained by the accelerometer, wherein the subject is in one or more postures during the activity event; (ii) determining at least one quality indicator related to the detected activity event; (iii) determining whether each of the at least one quality indicators meets a respective predetermined criterion; (iv) responsive to at least one quality indicator meeting the respective predetermined criterion, deciding whether information related to the detected activity event can be stored in the memory unit, and if so, storing information related to the detected activity event in the memory unit together with information related to one or more other activity events if any; (v) determining whether a calibration procedure should be performed based on information stored in the memory unit, and if so, processing the stored information to determine a transformation matrix, wherein the transformation matrix relates a frame of reference of the accelerometer to a frame of reference of the subject. Thus, this aspect provides for the activities of the subject to be monitored and activity events identified that might be suitable for determining the orientation of an accelerometer that is worn or carried by the subject relative to the subject. A transformation matrix determined from this/these events will provide a better estimate of the relationship between the frame of reference of the accelerometer and the frame of reference of the subject.

In some embodiments, the method further comprises a step (vi) after step (v) in which steps (i)-(v) are repeated for additional acceleration measurements obtained by the accelerometer. In this way, further suitable activity events can be detected, and used to refine the accuracy of the transformation matrix over time.

In some embodiments, repeating step (i) comprises detecting an activity event by the subject using acceleration measurements obtained by the accelerometer and the determined transformation matrix. In this way, the improved (i.e.

more accurate) transformation matrix is used in the activity detection phase of the method, thereby improving the detection of the activities.

In some embodiments, the at least one quality indicator is indicative of the suitability of the detected activity event for use in the calibration procedure. In these embodiments, the at least one quality indicator can comprise one or more of: an activity score; a duration of the detected activity event; and an indication of the consistency in an orientation between time windows.

In some embodiments, the method further comprises, responsive to each of the at least one quality indicators failing to meet the respective predetermined criterion, discarding information related to the detected activity event. In this way, unsuitable activity events are not used in the calibration procedure.

In some embodiments, the step of determining whether information related to the detected activity event can be stored in a memory unit comprises determining whether there is sufficient space in the memory unit to store information related to the detected activity event; responsive to determining that there is sufficient space, storing information related to the detected activity event in the memory unit; responsive to determining that there is insufficient space in the memory unit for storing the information, comparing the at least one quality indicator related to the detected activity event to corresponding quality indicators related to a plurality of previously detected activity events for which information is stored in the memory unit; determining whether to store information related to the detected activity event in place of stored information for a previously detected activity event based on the comparison, and if so, storing the storing information related to the detected activity event in the memory unit, otherwise discarding information related to the detected activity event. In this way the information stored in the memory unit is managed so that the most suitable information for performing the calibration procedure is retained in the memory unit.

In some embodiments, the stored information related to the detected activity event comprises one or more of (a) a mean gravity vector determined from the acceleration measurements related to the detected activity event; (b) the determined at least one quality indicator related to the detected activity event; (c) the acceleration measurements related to the detected activity event.

In some embodiments, step (v) comprises determining whether information related to at least a predetermined number of activity events has been stored in the memory unit.

In some embodiments, step (v) comprises determining whether the calibration procedure can be performed based on an amount of variation between respective mean gravity vectors for each activity event stored in the memory unit. In this way, the calibration procedure may only be performed if the data is consistent enough to produce a reliable result.

In some embodiments, the step of processing the stored information to determine the transformation matrix comprises determining the transformation matrix based on a mean gravity vector for each detected activity event stored in the memory unit.

In some embodiments, the method further comprises, responsive to determining that the calibration procedure cannot be performed, using a pre-defined transformation matrix to transform acceleration measurements between the frame of reference of the accelerometer and the frame of reference of the subject.

In some embodiments, the method further comprises using the determined transformation matrix to transform acceleration measurements between a frame of reference of the accelerometer and a frame of reference of the subject.

In some embodiments, the method further comprises responsive to determining that the calibration procedure cannot be performed in step (v), repeating steps (i)-(v) for additional acceleration measurements obtained by the accelerometer.

According to a second aspect, there is provided a computer program product comprising a computer readable medium, the computer readable medium having computer readable code embodied therein, the computer readable code being configured such that, on execution by a suitable computer or processor, the computer or processor is caused to perform the method according to the first aspect, or any embodiment thereof.

According to a third aspect, there is provided an apparatus to determine a transformation matrix for measurements from an accelerometer that is worn by a subject. The apparatus comprises a memory unit arranged to store information for several activity events, and the apparatus further comprises a processing unit that is coupled to the memory unit, with the processing unit being configured to (i) detect an activity event in acceleration measurements obtained by the accelerometer (where the subject is in one or more postures during the activity event); (ii) determine at least one quality indicator related to the detected activity event; (iii) determine whether each of the at least one quality indicators meets a respective predetermined criterion; (iv) responsive to at least one quality indicator meeting the respective predetermined criterion, decide whether information related to the detected activity event can be stored in the memory unit, and if so, store information related to the detected activity event in the memory unit together with information related to one or more other activity events if any; (v) determine whether a calibration procedure should be performed based on information stored in the memory unit, and if so, process the stored information to determine a transformation matrix, wherein the transformation matrix relates a frame of reference of the accelerometer to a frame of reference of the subject. Thus, this aspect provides for the activities of the subject to be monitored and activity events identified that might be suitable for determining the orientation of an accelerometer that is worn or carried by the subject relative to the subject. A transformation matrix determined from this/these events will provide a better estimate of the relationship between the frame of reference of the accelerometer and the frame of reference of the subject.

In some embodiments, the processing unit is further configured to repeat operations (i)-(v) for additional acceleration measurements obtained by the accelerometer. In this way, further suitable activity events can be detected and used to refine the accuracy of the transformation matrix over time.

In some embodiments, repeating operation (i) comprises detecting an activity event by the subject using acceleration measurements obtained by the accelerometer and the determined transformation matrix. In this way, the improved (i.e. more accurate) transformation matrix is used in the activity detection phase of the method, thereby improving the detection of the activities.

In some embodiments, the at least one quality indicator is indicative of the suitability of the detected activity event for use in the calibration procedure. In these embodiments, the at least one quality indicator can comprise one or more of: an activity score; a duration of the detected activity event; and an indication of the consistency in an orientation between time windows.

In some embodiments, the processing unit is further configured to, responsive to each of the at least one quality indicators failing to meet the respective predetermined criterion, discard information related to the detected activity event. In this way, unsuitable activity events are not used in the calibration procedure.

In some embodiments, the processing unit is configured to determine whether information related to the detected activity event can be stored in a memory unit by determining whether there is sufficient space in the memory unit to store information related to the detected activity event; responsive to determining that there is sufficient space, storing information related to the detected activity event in the memory unit; responsive to determining that there is insufficient space in the memory unit for storing the information, comparing the at least one quality indicator related to the detected activity event to corresponding quality indicators related to a plurality of previously detected activity events for which information is stored in the memory unit; determining whether to store information related to the detected activity event in place of stored information for a previously detected activity event based on the comparison, and if so, storing the storing information related to the detected activity event in the memory unit, otherwise discarding information related to the detected activity event. In this way the information stored in the memory unit is managed so that the most suitable information for performing the calibration procedure is retained in the memory unit.

In some embodiments, the stored information related to the detected activity event comprises one or more of (a) a mean gravity vector determined from the acceleration measurements related to the detected activity event; (b) the determined at least one quality indicator related to the detected activity event; (c) the acceleration measurements related to the detected activity event.

In some embodiments, operation (v) comprises determining whether information related to at least a predetermined number of activity events has been stored in the memory unit.

In some embodiments, operation (v) comprises determining whether the calibration procedure can be performed based on an amount of variation between respective mean gravity vectors for each activity event stored in the memory unit. In this way, the calibration procedure may only be performed if the data is consistent enough to produce a reliable result.

In some embodiments, the processing unit is configured to process the stored information to determine the transformation matrix based on a mean gravity vector for each detected activity event stored in the memory unit.

In some embodiments, the processing unit is further configured to, responsive to determining that the calibration procedure cannot be performed, use a pre-defined transformation matrix to transform acceleration measurements between the frame of reference of the accelerometer and the frame of reference of the subject.

In some embodiments, the processing unit is further configured to use the determined transformation matrix to transform acceleration measurements between a frame of reference of the accelerometer and a frame of reference of the subject.

In some embodiments, the processing unit is further configured to, responsive to determining that the calibration procedure cannot be performed in operation (v), repeat operations (i)-(v) for additional acceleration measurements obtained by the accelerometer.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 is a block diagram illustrating an apparatus according to an exemplary embodiment;

Fig. 2 is a flow chart illustrating a method according to an exemplary embodiment;
Fig. 3 is a flow chart illustrating a method according to another exemplary embodiment;

DETAILED DESCRIPTION OF EMBODIMENTS

As noted above, the invention aims to provide an improved technique for determining a transformation matrix that relates a frame of reference of an accelerometer to a frame of reference of a subject that is carrying or wearing the accelerometer.

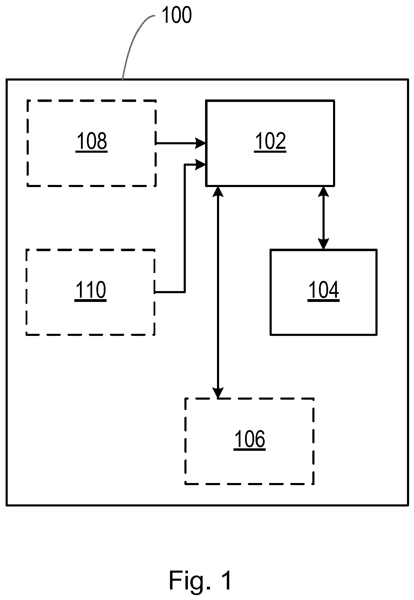

Fig 1 illustrates an apparatus 100 according to an embodiment. The apparatus 100 includes a processing unit 102 that controls the operation of the apparatus 100 and that can be configured to execute or perform the methods described herein. For example, the processing unit 102 can be used to process acceleration measurements obtained by an accelerometer. The acceleration measurements may be used to determine a transformation matrix. The processing unit 102 can be implemented in numerous ways, with software and/or hardware, to perform the various functions described herein. The processing unit 102 may comprise one or more microprocessors or digital signal processor (DSPs) that may be programmed using software or computer program code to perform the required functions and/or to control components of the processing unit 102 to effect the required functions. The processing unit 102 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The processing unit 102 is connected to a memory unit 104 that can store data, information and/or signals for use by the processing unit 102 in controlling the operation of the apparatus 100 and/or in executing or performing the methods described herein. For example, the memory unit 104 may store acceleration measurements obtained from an accelerometer and/or information derived from acceleration measurements, such as a transformation matrix. In some implementations the memory unit 104 stores computer-readable code that can be executed by the processing unit 102 so that the processing unit 102 performs one or more functions, including the methods described herein. The memory unit 104 can comprise any type of non-transitory machine-readable medium, such as cache or system memory including volatile and non-volatile computer memory such as random access memory (RAM) static RAM (SRAM), dynamic RAM (DRAM), read-only memory (ROM), programmable ROM (PROM), erasable PROM (EPROM) and electrically erasable PROM (EEPROM), implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

The apparatus 100 may also include interface circuitry 106 for enabling a data connection to and/or data exchange with other devices, including any one or more of servers, databases, user devices, and sensors. For example, where the accelerometer is separate from the apparatus 100, the interface circuitry 106 may be used to receive acceleration measurements obtained from the accelerometer. The connection may be direct or indirect (e.g. via the Internet), and thus the interface circuitry 106 can enable a connection between the apparatus 100 and a network, such as the Internet, via any desirable wired or wireless communication protocol. For example, the interface circuitry 106 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 106 (and thus apparatus 100) may include one or more suitable antennas for transmitting/receiving over a transmission medium (e.g. the air). Alternatively, in the case of a wireless connection, the interface circuitry 106 may include means (e.g. a connector or plug) to enable the interface circuitry 106 to be connected to one or more suitable antennas external to the apparatus 100 for transmitting/receiving over a transmission medium (e.g. the air). The interface circuitry 106 is connected to the processing unit 102.

In general, the apparatus 100 can be any type of electronic device or computing device. In some implementations, for example as shown in Fig. 1, the apparatus 100 is an apparatus that is carried or worn by the user. For example, the apparatus 100 can be, or be part of, a laptop, a tablet, a smartphone, a smartwatch, etc., or be integrated into an item of clothing (e.g. a shirt, trousers, a coat, etc.) or be in the form of a wearable accessory (e.g. a wrist band, a pendant (including a Personal Help Button (PHB) that has a button that a user can push to summon help in an emergency), a necklace, a chest band, etc.). In other implementations, the apparatus 100 is an apparatus that is present or used in the home or care environment of the user. For example, the apparatus 100 can be, or be part of, a laptop, a tablet, a smartphone or a computer. In other implementations, the apparatus 100 is an apparatus that is remote from the user, and remote from the home or care environment of the user. For example, the apparatus 100 can be a server, for example a server in a data center (also referred to as being 'in the cloud').

As noted above, the apparatus 100 determines a transformation matrix that relates a frame of reference of an accelerometer that is worn or carried by a subject to a frame of reference of the subject. Thus, the apparatus 100 requires measurements from an accelerometer (referred to herein as 'acceleration measurements') that is worn or carried by the subject. In implementations where the apparatus 100 is carried or worn by the user, the apparatus 100 can comprise an accelerometer 108 that measures accelerations as the apparatus 100 is carried or worn by the user. The accelerometer 108 is connected or coupled to the processing unit 102 (and/or to the memory unit 104).

It will be appreciated that although the accelerometer 108 is shown as part of the apparatus 100 in Fig. 1, in other implementations, the accelerometer 108 may be separate from the part of the apparatus 100 that includes the processing unit 102 (for example in a separate housing or body), and the accelerometer 108 may be connected using a wired connection or wirelessly to the rest of the monitoring apparatus 100, including the processing unit 102 (e.g. via the interface circuitry 106). For example the accelerometer 108 may be part of a smart watch or pendant (including a PHB), and the processing unit 102 can be part of a smart phone to which the smart watch or pendant is paired.

In alternative implementations to that shown in Fig. 1, for example where the apparatus 100 is not in a form that can be carried or worn by the user, an accelerometer can be provided separately from apparatus 100, and accelerometer can be provided in a housing or body that can be worn or carried by the user (e.g. in the form of a pendant (including a PHB), necklace, watch, bracelet, wrist band, chest band or chest strap, etc.). In that case, the accelerometer may have interface circuitry associated therewith that enables the communication of the acceleration measurements to the processing unit 102 in the apparatus 100.

Regardless of the implementation of the apparatus 100 and the location of the accelerometer 108 with respect to the apparatus 100, the accelerometer 108 can generate a measurement signal that contains a plurality of acceleration measurement samples representing the movements of the user at a plurality of time instants. The accelerometer 108 is typically an accelerometer that measures accelerations in three dimensions, and the measurement signal generated by the accelerometer 108 can include respective measurement signals representing the accelerations in each of the three dimensions. For example, the accelerometer 108 can output respective measurement signals for each of an x-axis, y-axis and z-axis of a Cartesian coordinate system.

It will be appreciated that a practical implementation of an apparatus 100 may include additional components to those shown in Fig. 1. For example the apparatus 100 may also include a power supply, such as a battery, or components for enabling the apparatus 100 to be connected to a mains power supply.

As another example, the apparatus 100 may comprise a user interface 110 that includes one or more components that enables a user of apparatus 100 (who could be the subject or any other person) to input information, data and/or commands into the apparatus 100, and/or enables the apparatus 100 to output information or data to the user of the apparatus 100. The user interface 110 can comprise any suitable input component(s), including but not limited to a keyboard, keypad, one or more buttons, switches or dials, a mouse, a track pad, a touchscreen, a stylus, a camera, a microphone, etc., and the user interface 110 can comprise any suitable output component(s), including but not limited to a display screen, one or more lights or light elements, one or more loudspeakers, a vibrating element, etc.

As noted above, the orientation of the accelerometer 108 with respect to the subject may not initially be known, and the orientation of the accelerometer with respect to the subject may change over time. The subject's frame of reference can be represented by three orthogonal 'body' axes (labelled xb, yb and zb to distinguish from the accelerometer measurement axes) which can be defined relative to a part of the body, such as the torso or chest. For example, yb can be defined as pointing from the torso upwards towards the head, xb can be defined as pointing from the center of the torso to the left arm, and zb can be orthogonal to the other two axes (e.g. pointing forwards from the torso). However, it will be appreciated that the choice of the reference systems and the choice of the orientation of their axes are arbitrary.

To determine the posture of the subject (for example) from the acceleration measurements, a calibration procedure is required as it is not known how the acceleration measurements (for example represented in an x, y and z reference frame of the accelerometer 108), relate to the subject's frame of reference. Thus, the calibration procedure determines a transformation matrix that enables the accelerations measured in the frame of reference of the accelerometer 108 to be transformed into the body axes system (xb, yb, zb).

Briefly, the invention provides improved techniques for determining the transformation matrix in which the activity of the subject is monitored and activity events are identified that might be suitable for calibrating (i.e. determining) the orientation of an accelerometer that is worn or carried by the subject. When sufficient events have been identified and stored, a calibration procedure is performed to determine the transformation matrix. In some embodiments, further activity event(s) are identified that might improve the accuracy of the calibration procedure, and the calibration procedure can be repeated based on the further activity event(s) to refine or improve the accuracy of the transformation matrix.

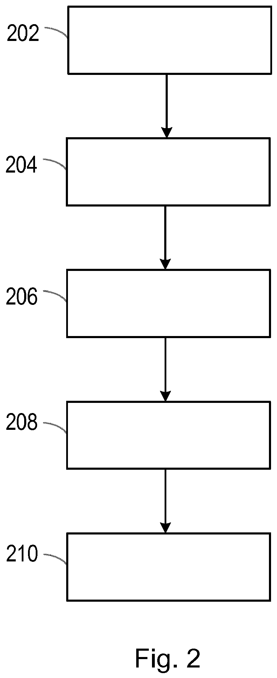

The flow chart in Fig. 2 illustrates an exemplary method according to the techniques described herein. One or more of the steps of the method can be performed by the processing unit 102 in the apparatus 100, in conjunction with any of the memory unit 104, interface circuitry 106 (if present), accelerometer 108 and user interface 110 (if present) as appropriate. The processing unit 102 may perform the one or more steps in response to executing computer program code, that can be stored on a computer readable medium, such as, for example, the memory unit 104.

In a first step, step 202, the processing unit 102 detects an activity event in acceleration measurements obtained by the accelerometer 108. The activity event detected can be any activity event in which the subject is in one or more postures during the activity event. In other words, each respective activity event has an associated one or more postures. For example, the activity event may be walking, and it is known that the subject will be in an upright posture (or series of upright postures) during the walking event. It will be appreciated that postures of the subject have respective orientations with respect to gravity (for example, when the subject is in an upright posture, gravity will be generally acting in the negative direction of the yb axis defined above). Examples of types of activity event that may be detected include (but are not limited to) standing, walking, jogging, running or cycling. A detected activity event will have a determined activity start time to a determined activity end time.

In some embodiments, step 202 can comprise detecting only one particular type of activity event (for example walking), but in other embodiments, step 202 can comprise detecting more than one particular type of activity event (for example walking and cycling). Where more than one type of activity event can be detected, the different types of activity event are preferably such that the subject is in a common posture or postures (e.g. the subject is upright in each of the activity events).

It will be appreciated that as the orientation of the accelerometer 108 with respect to the reference frame of the subject may not be known (or known with sufficient accuracy) at this stage, the activity event that is to be detected in step 202 should be detectable without needing to know (or knowing accurately) the orientation of the accelerometer 108 with respect to the subject.

The processing unit 102 can detect an activity event in acceleration measurements obtained by the accelerometer 108 by processing the acceleration measurements in a number of different ways, and generally, those skilled in the art will be aware of numerous techniques for identifying the types of activity events mentioned above.

In some embodiments, the processing unit 102 may detect an activity event in acceleration measurements obtained by the accelerometer 108 by use of a logistic regression model. In some embodiments, the logistic regression model may be applied to the acceleration measurements obtained by the accelerometer 108 over a particular length of time (for example, five seconds), and log-odds determined for whether the portion of acceleration measurements relate to the desired activity event or not. In one example, where a walking event is being detected, the log-odds may relate to whether the measurements are a "walking activity" or a "non-walking activity". In other embodiments, the processing unit 102 may detect an activity event in acceleration measurements obtained by the accelerometer 108 by use of alternative types of classifier such as a support vector machine (SVM), a decision tree, xgboost, a neural network, etc.

Next, in step 204, the processing unit 102 determines at least one quality indicator related to the detected activity event. In subsequent steps of the method, the at least one quality indicator is used to determine the suitability of the acceleration measurements relating to the detected activity event for use in a calibration procedure, and thus any type of quality indicator can be used that serves this purpose.

In some embodiments, the at least one quality indicator comprises one or more of an activity score, such as a mean activity score (or walking score or mean walking score if the activity event is walking), a duration of the detected activity event (for example measured in seconds) and an indication of the consistency in an orientation between time windows (for example measured in radians or degrees).

In some embodiments, the or each quality indicator can be expressed according to their normal or typical units (for example seconds for duration and radians for the consistency in orientation). In alternative embodiments, each quality indicator can be determined in the form of a score (also referred to herein as a quality indicator score), which can make it easier to compare the values of the different quality indicators to each other.

Where the at least one quality indicator comprises a duration of the detected activity event, the duration of the detected activity event can be determined from the timing of the first acceleration measurement(s) at which the activity event is detected and the last acceleration measurement(s) at which the activity event is detected. The duration is typically expressed in seconds or another unit of time, but in some embodiments the duration of the activity event can be converted to a score. As one example, the duration score ($D_{score}$) may be computed as follows:

$$D_{score} = (D - 15) / 0.15, \text{ where } D \le 30; \text{ and}$$

$$D_{score} = 200 - (3000 / D), \text{ where } D > 30, \tag{1}$$

where D is the duration of the detected activity event in seconds. It will be appreciated that this example means that $D_{score}$ will be less than 0 if the duration is less than 15 seconds, and greater than 0 if the duration is greater than 15 seconds. In other examples the duration that leads to the 'zero-crossing' of the duration score may be different than 15 seconds.

Where the quality indicator is the activity score, a mean activity score may be determined as the average value (e.g. mean, median or mode) of the log-odds (as described above) determined for a plurality of time windows within a detected activity event. Alternatively, the activity score can be based on the periodicity and/or signal power of the acceleration measurements relating to the activity event. In embodiments where an alternative type of classifier is used to detect an activity event, the activity score can be based on an output of the classifier.

To make it easier to compare the (mean) activity score to the scores of other quality indicators, the activity score can be 'normalised'. For example a normalised mean activity score ($MAS_{normalised}$) may be computed as follows:

$$MAS_{normalised} = (MAS - 10) / 0.3 \qquad (2)$$

where MAS is the (un-normalised) mean activity score. It will be appreciated that this example means that $MAS_{normalised}$ will be less than 0 if the mean activity score is less than 10, and the $MAS_{normalised}$ will be more than 0 if the MAS is greater than 10. In other examples the MAS that leads to the 'zero-crossing' of the normalised MAS may be different than 10.

Where the at least one quality indicator is or includes an indication of the consistency in an orientation between time windows, the indication of the consistency in an orientation between time windows may be computed as follows.

For the detected activity event, a unit vector representing the direction of the mean acceleration vector is determined from the acceleration measurements obtained by the accelerometer 108 in one or more time windows (e.g. of a duration of 5 seconds) corresponding to the activity event. Thus, a unit vector representing the direction of the mean acceleration vector is obtained for each time window. The unit vector representing the direction of the mean acceleration vector for each window may be determined by dividing the 3D mean of the acceleration vectors for that window by the vector magnitude of the 3D mean of the acceleration vectors for that window. The direction of the unit vector can be assumed to be equivalent to the vertical direction in the accelerometer reference frame. The variances of the unit vector may then be determined in one or more of the x-direction, the y-direction and the z-direction (in the reference frame of the accelerometer 108). For example the variance in the x-direction is the variance of the x-elements of the 3D unit vectors for the windows. The square root of the sum of the variance in the x-direction, the variance in the y-direction and the z-direction may then be computed. This value is used as the standard deviation of the vertical direction in the accelerometer 108 reference frame, which can also be understood as being equivalent to the standard deviation of the inclination or the orientation of the accelerometer 108 with respect to gravity.

The standard deviation of the vertical direction in the accelerometer reference frame is a measure of consistency (or inconsistency) of the orientation of the accelerometer 108 over time.

It will be appreciated that where the accelerometer 108 is fixed to the body of the subject, any variation of the inclination or the orientation of the accelerometer 108 with respect to gravity will correspond to a variation of the inclination or the orientation of the body of the subject with equal magnitude. Thus, the consistency in the accelerometer 108 orientation will be equivalent to the consistency in the body orientation of the subject.

The indication of the consistency in orientation between time windows can typically be expressed in degrees or radians, but in some embodiments the indication can be converted to a score. As one example, the orientation consistency score, $C_{score}$, may be computed as follows:

$$C_{score} = 1.25 / (C + 0.005) - 25 \qquad (3)$$

where C is the unit vector standard deviation in radians. It will be appreciated that in this example means that $C_{score}$ will be less than 0 if the unit vector standard deviation is more than 0.045 radians, and greater than 0 if the unit vector standard deviation is less than 0.045 radians. In other examples the unit vector standard deviation that leads to the 'zero-crossing' of the $C_{score}$ may be different than 0.045 radians.

In step 206, the processing unit 102 determines whether each of the at least one quality indicators meets a respective predetermined criterion. Step 206 aims to identify detected activities that are suitable for use in a calibration procedure, and thus the predetermined criterion relates to the suitability of the detected activity event (as represented by the quality indicator(s)) for use in calibration.

For the duration of the detected activity event, a longer duration may be indicative that the detected activity event is more suitable for use in a calibration procedure than say, a shorter duration. Thus, the predetermined criterion for the activity duration may be a threshold, with the criterion being met if the activity duration (or activity duration score) is above the threshold.

In some examples, the predetermined criterion for the activity duration may be a threshold with a value of 15 seconds, so if the duration of the detected activity event is 15 seconds or more, then the criterion may be met. If the activity duration is converted to a score as described above with reference to equation (1), the threshold may be 0, so if $D_{score}$ is equal to or greater than 0, the criterion is met. As an example, although a duration of 15 seconds may be considered suitable for use in calibration, an activity event with a duration of, say, 30 seconds, may be considered particularly suitable for use in calibration.

For the activity score, a higher value may be indicative that the detected activity event is more suitable for use in a calibration procedure. In particular, the greater the average value of the log-odds determined for a plurality of windows for a detected activity event, the more likely that the subject is actually performing or executing the activity, and thus the more likely that the detected activity event will be suitable for use in a calibration procedure.

In some examples, the predetermined criterion for the activity score may be a threshold, with the criterion being met if the activity score (or normalised activity score) is above the threshold.

In some examples, the predetermined criterion for the activity score may be a threshold with a value of 10, so if the activity score for the detected activity event is 10 or more, then the criterion may be met. If the activity score is normalised as described above with reference to equation (2), the threshold may be 0, so if $MAS_{normalised}$ is equal to or greater than 0, the criterion is met. As an example, although a MAS of 10 may be considered suitable for use in calibration, an activity event with a MAS of, say, 40, may be considered particularly suitable for use in calibration.

For the orientation consistency, a lower value may be indicative that the detected activity event is more suitable for use in a calibration procedure. Thus, the predetermined criterion for the orientation consistency may be a threshold, with the criterion being met if the orientation consistency (or orientation consistency score) is below the threshold.

In some examples, the predetermined criterion for the orientation consistency may be a threshold with a value of 0.045 radians, so if the unit vector standard deviation is 0.045 or less, the criterion is met and the quality indicator indicates that the detected activity event is of sufficient quality for use in a calibration procedure. If the orientation consistency is converted to a score as described above with reference to equation (3), the threshold may be 0, so if $C_{score}$ is equal to or less than 0, the criterion is met. As an example, although an orientation consistency of 0.045 radians may be considered suitable for use in calibration, an orientation consistency with a value of, say, 0.005 radians, may be considered particularly suitable for use in calibration.

In step 208, responsive to at least one quality indicator meeting the respective predetermined criterion, the processing unit 102 decides whether information related to the detected activity event can be stored in the memory unit 104, and if so, information related to the detected activity event is stored in the memory unit 104. The memory unit 104 may already store information relating to one or more other previously-detected activity events, in which case the information on the activity event detected in step 202 is stored in the memory unit 104 together with (i.e. alongside) the, or some of the, information for the other activity events.

If none of the at least one quality indicators meets the respective predetermined criterion, or if the processing unit 102 decides that information for the detected activity event should not be stored, the processing unit 102 discards the detected activity event and returns to step 202 for the next activity event that is detected in the acceleration measurements.

The processing unit 102 may decide whether information related to the detected activity event can be stored in the memory unit 104 based on the amount of available storage or space within the memory unit 104 and/or based on the at least one quality indicator.

Thus, in some embodiments, the processing unit 102 determines whether there is sufficient space in the memory unit 104 to store information related to the detected activity event, and responsive to determining that there is sufficient space, the processing unit 102 stores information related to the detected activity event in the memory unit 104. For example, the memory unit 104 may be configured to store information related to a predefined number of detected activity events (for example 10), and the processing unit 102 may determine that the memory unit 104 is presently storing information related to a number of detected activity events that is less than this predefined number.

If the processing unit 102 determines that there is insufficient space in the memory unit 104 for storing the information (e.g. the memory unit 104 is full, or the portion of the memory unit 104 allocated to storing information about detected activity events is full, or the memory unit 104 is already storing information about the predefined number of activity events), the processing unit 102 compares quality indicator(s) related to the activity event detected in step 202 to corresponding quality indicators for the activity events already stored in the memory unit 104. For example, the processing unit 102 may compare one or more of: the (mean) activity score of the detected activity event to the (mean) activity scores for the stored activity events, the duration of the detected activity event to the durations for the stored activity events, and the orientation consistency of the detected activity event to the orientation consistency for the stored activity events.

The processing unit 102 can then determine whether to store information related to the detected activity event in place of stored information for a previously detected activity event based on the comparison, and if so, the processing unit 102 stores information related to the detected activity event in the memory unit 104 in place of the information for a previously detected activity event. If it is determined that the processing unit 102 should not store information about the activity event detected in step 202, the processing unit 102 discards the detected activity event and the method returns to step 202 and repeats for the next activity event that is detected in the acceleration measurements.

For example, if the comparison indicates that the quality indicators for the detected activity event are higher than one or more of (or each of) the quality indicators for one or more of the activity events stored in the memory unit 104, the processing unit 102 can determine that the detected activity event can be stored in the memory unit 104 in place of the stored activity event with the lower quality indicator. As another example, if the comparison indicates that the minimum value of the quality indicator scores for the detected activity event is higher than the minimum value of the quality indicator scores for one or more of the stored activity events the processing unit 102 can determine that the detected activity event can be stored in the memory unit 104 in place of the stored activity event with the lower quality indicator score.

In other words, until the memory unit 104 can no longer store additional information related to detected activity events, the processing unit 102 may simply store information about a detected activity event where it is found that the detected activity event is of sufficient quality for use in a calibration procedure (i.e. at least one quality indicator meets the respective predetermined criterion). Once the memory unit 104 is full, the processing unit 102 may give precedence to a detected activity event where it is found that the detected activity event is better than a stored activity event for calibration. Those skilled in the art will be aware of various ways in which it can be determined whether to store information related to the detected activity event in place of stored information for a previously detected activity event based on a comparison of the quality indicators.

In some embodiments, the information related to the detected activity event to be stored in the memory unit 104 (and the information already stored in the memory unit 104 for previous activity events) may comprise one or more of a mean gravity vector (which can be a unit vector) determined from the acceleration measurements related to the detected activity event (i.e. a vector which represents the direction in which gravity is acting over the activity event), the determined at least one quality indicator related to the detected activity event, and the acceleration measurements related to the detected activity event. In embodiments where step 202 can comprise detecting more than one type of activity event (for example walking and cycling), the information related to the activity event to be stored in the memory unit 104 can include an identification of the type of activity event detected, and/or the type of posture involved in the activity event.

In step 210, the processing unit 102 determines whether a calibration procedure should be performed based on the information stored in the memory unit 104, and if so, the processing unit 102 processes the stored information to determine a transformation matrix. As noted above, the transformation matrix relates the frame of reference of the accelerometer 108 to the frame of reference of the subject. If it is determined that a calibration procedure should not be performed, the method can return to step 202 and repeat for the next activity event that is detected in the acceleration measurements.

In some embodiments, step 210 can comprise determining that a calibration procedure should be performed if the memory unit 104 contains information relating to at least a minimum number of activity events. For example, it may be necessary for the memory unit 104 to contain information relating to at least 6 previously detected activity events in order for a calibration procedure to be performed (in other words, there is enough data for a calibration procedure to be performed).

Additionally or alternatively, in some embodiments, step 210 can comprise determining that a calibration procedure can be performed if the variation between the mean gravity unit vectors for the stored activity events is below a predefined threshold (in other words, the data is consistent enough for a calibration procedure to be performed). The variation can be determined as the square root of the sum of the unit vector variances. For example, the calibration procedure can be performed if the variation in the mean gravity directions is less than 0.03.

In some embodiments, information for all of the stored activity events can be used to determine the transformation matrix. In other embodiments, information related to a particular subset of the activity events can be used to determine the transformation matrix. For example, the stored information related to a particular percentage of the previously detected activity events can be processed, for example, 60%, or the 'middle' 60% of the gravity vectors can be processed. It will be appreciated that a particular subset of previously detected activity events may also be selected based on other factors, for example, the activity events with the highest quality indicator(s).

The step of processing the stored information to determine a transformation matrix may comprise averaging the respective mean gravity direction unit vectors of the required activity events (e.g. all of the activity events or a subset) to give an average gravity direction. The transformation matrix is determined using the average gravity direction to enable rotations between the accelerometer reference frame and the subject reference frame.

In some embodiments, the method may further comprise using the determined transformation matrix to relate acceleration measurements in the frame of reference of the accelerometer 108 to the frame of reference of the subject. For example, the transformation matrix may be used to transform acceleration measurements to the reference frame of the subject. As another example, the transformation matrix may be used to transform a set of thresholds (expressed in the subject's frame of reference) that are used to identify postures of the subject into the reference frame of the accelerometer 108.

In some embodiments, before the method in Fig. 2 is performed, a predefined transformation matrix may be provided, and the method in Fig. 2 can be used to update or replace the predefined transformation matrix. Until the predefined transformation matrix is updated or replaced, it can be used for transform between the accelerometer reference frame and the subject reference frame. In some embodiments, the predefined transformation matrix can be used in step 202 to detect an activity event. The predefined transformation matrix may be defined based on an assumption of the orientation and/or inclination of the accelerometer 108 with respect to the subject. For example, the pre-defined transformation matrix may be selected based on an assumption that the accelerometer 108 is worn at the trunk of the subject, with the y-axis of the accelerometer 108 aligned with the yb-axis of the subject.

After performing the calibration procedure, the processing unit 102 may repeat steps 202 to 210 for additional acceleration measurements obtained by the accelerometer 108 to determine an updated transformation matrix.

In some embodiments, the repetition of step 202 may comprise detecting an activity event by the subject using acceleration measurements obtained by the accelerometer 108 and the determined transformation matrix. Thus, the activity event is more likely to be accurately and/or reliably detected in acceleration measurements obtained by the accelerometer 108, as the acceleration measurements obtained by the accelerometer 108 have been evaluated using a more recent and more accurate transformation matrix.

As noted above, step 202 can comprise detecting only one particular type of activity event (for example walking), but in other embodiments, step 202 can comprise detecting more than one particular type of activity event (for example walking and cycling), and these multiple types of activity event are considered together when performing the calibration procedure.

In an alternative implementation, the method in Fig. 2 can (in effect) be performed separately for different types of activity event to determine respective transformation matrices from the information for those activity events. In these embodiments, each instance of the method in Fig. 2 can store the information for the relevant detected activity event(s) in a respective memory unit or in respective parts of the memory unit 104. The determined transformation matrices can then be used for detecting that particular type of activity event (i.e. a transformation matrix derived from detected walking activity events can be used to detect walking events in further acceleration measurements, etc.), or those transformation matrices can be combined or compared to determine a transformation matrix to use for evaluating further acceleration measurements. The multiple transformation matrices can be combined to form an overall transformation matrix (e.g. by averaging the matrices for the different activity events). Alternatively, the multiple transformation matrices can be checked for consistency with each other and one or both used to evaluate further acceleration measurements.

For example, the acceleration measurements can be analyzed by a first instance of the method in Fig. 2 to detect walking events, and the same acceleration measurements can be analyzed by a second instance of the method in Fig. 2 to detect cycling events. The information for the detected walking events can be stored separately from the information for the detected cycling events (for example in respective memory units or in respective parts of the memory unit 104). The calibration procedure in each instance of the method in Fig. 2 will make use of the stored information for the relevant type of activity event to determine a respective transformation matrix.

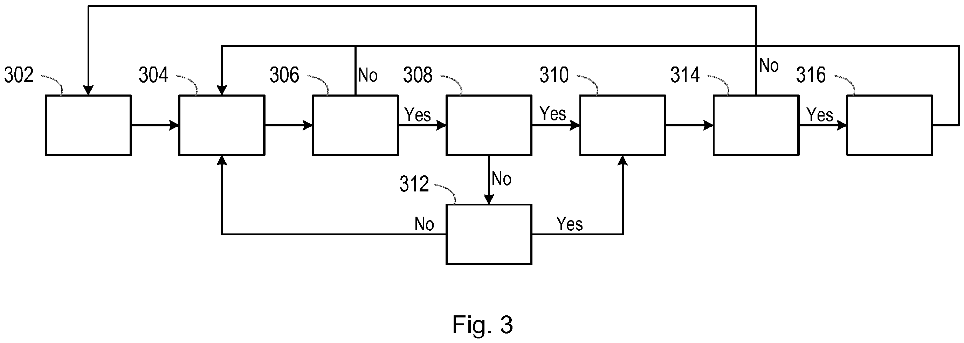

The flow chart in Fig. 3 illustrates a specific method according to the techniques described herein. One or more of the steps of the method can be performed by the processing unit 102 in the apparatus 100, in conjunction with any of the memory unit 104, interface circuitry 106 (if present), accelerometer 108 and user interface 110 (if present) as appropriate. The processing unit 102 may perform the one or more steps in response to executing computer program code, that can be stored on a computer readable medium, such as, for example, the memory unit 104.

The method in Fig. 3 relates to detecting walking events, but it will be appreciated that the method can be applied to other types of activity event.

In step 302, the processing unit 102 uses a predefined transformation matrix to relate acceleration measurements in the frame of the accelerometer 108 to the frame of reference of the subject.

In step 304, the processing unit 102 detects a walking event in acceleration measurements obtained by the accelerometer 108. It will be appreciated that step 304 may be performed in a similar manner as step 202, as described above.

In step 306, the processing unit 102 determines at least one quality indicator related to the detected walking event and a respective quality indicator score, and determines whether each of the at least one quality indicator scores is greater than 0.

If at least one quality indicator score is less than 0, the processing unit 102 discards the detected activity event, and the method returns to step 304. It will be appreciated that step 306 may be performed in a similar manner as steps 204 and 206, as described above.

If all of the quality indicators are greater than 0, then the method proceeds to step 308. At step 308, the processing unit 102 determines whether there is sufficient space in the memory unit 104 to store information related to the detected walking event. If there is sufficient space, the method proceeds to step 310. At step 310, the processing unit 102 stores information related to the detected walking event in the memory unit 104, and in particular the quality indicator scores for the walking event and a mean gravity direction unit vector for the walking event.

If at step 308 there is insufficient space in the memory unit 104 for storing the information, the method proceeds to step 312. At step 312, the processing unit 102 determines whether the minimum quality indicator score for the detected walking event is higher than the minimum quality indicator score for any walking event already stored in the memory unit 104. If so, the information related to the detected activity event is stored in place of the stored information for a previously detected walking event with a lower minimum quality indicator score (step 310). If the minimum quality indicator score for the detected walking event is not higher than the minimum quality indicator score for any walking event already stored in the memory unit 104, the processing unit 102 discards information related to the detected walking event, and the method returns to step 304. It will be appreciated that steps 308, 310 and 312 maybe performed in a similar manner as step 208, as described above.

Following step 310, the method proceeds to step 314. At step 314, the processing unit 102 determines whether a calibration procedure should be performed based on the information stored in the memory unit 104. If the processing unit 102 determines that a calibration procedure should be performed based on information stored in the memory unit 104, the method proceeds to step 316. At step 316, the processing unit 102 processes the stored information to determine a transformation matrix, wherein the transformation matrix relates a frame of reference of the accelerometer 108 to a frame of reference of the subject.

If, at step 314, the processing unit 102 determines that a calibration procedure should not be performed based on information stored in the memory unit 104, the method returns to step 302, and the processing unit 102 uses the pre-defined transformation matrix to transform acceleration measurements between the frame of reference of the accelerometer 108 and the frame of reference of the subject. It will be appreciated that steps 314 and 316 may be performed in a similar manner as step 210, as described above.

There is therefore provided an improved method and apparatus for determining a transformation matrix that relates a frame of reference of the accelerometer to a frame of reference of the subject.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method of operating an apparatus to determine a transformation matrix for measurements from an accelerometer that is worn by a subject, the apparatus comprising a memory unit arranged to store information for several activity events, the method comprising:

   (i) detecting (202) an activity event in acceleration measurements obtained by the accelerometer, wherein the subject is in one or more postures during the activity event;
   (ii) determining (204) at least one quality indicator related to the detected activity event;
   (iii) determining (206) whether each of the at least one quality indicators meets a respective predetermined criterion;
   (iv) responsive to at least one quality indicator meeting the respective predetermined criterion, deciding (208) whether information related to the detected activity event can be stored in the memory unit, and if so, storing information related to the detected activity event in the memory unit together with information related to one or more other activity events if any;
   (v) determining (210) whether a calibration procedure should be performed based on information stored in the memory unit, and if so, processing the stored information to determine a transformation matrix, wherein the transformation matrix relates a frame of reference of the accelerometer to a frame of reference of the subject.

2. A method according to claim 1, wherein the method further comprises:

   (vi) after step (v), repeating steps (i)-(v) for additional acceleration measurements obtained by the accelerometer.

3. A method according to claim 2, wherein repeating step (i) comprises:

   detecting an activity event by the subject using acceleration measurements obtained by the accelerometer and the determined transformation matrix.

4. A method according to any preceding claim, wherein the at least one quality indicator is indicative of the suitability of the detected activity event for use in the calibration procedure.

5. A method according to any preceding claim, wherein the step of deciding (208) whether information related to the detected activity event can be stored in a memory unit comprises determining whether there is sufficient space in the memory unit to store information related to the detected activity event;
responsive to determining that there is sufficient space, storing information related to the detected activity event in the memory unit;
responsive to determining that there is insufficient space in the memory unit for storing the information, comparing the at least one quality indicator related to the detected activity event to corresponding quality indicators related to a plurality of previously detected activity events for which information is stored in the memory unit;
determining whether to store information related to the detected activity event in place of stored information for a previously detected activity event based on the comparison, and if so, storing the storing information related to the detected activity event in the memory unit, otherwise discarding information related to the detected activity event.

6. A method according to any preceding claim, wherein step (v) comprises determining whether information related to at least a predetermined number of activity events has been stored in the memory unit.

7. A method according to any preceding claim, wherein step (v) comprises determining whether the calibration procedure can be performed based on an amount of variation between respective mean gravity vectors for each activity event stored in the memory unit.

8. A computer program product comprising a computer readable medium, the computer readable medium having computer readable code embodied therein, the computer readable code being configured such that, on execution by a suitable computer or processor, the computer or processor is caused to perform the method of any of claims 1-7.

9. An apparatus (100) to determine a transformation matrix for measurements from an accelerometer (108) that is worn by a subject, the apparatus (100) comprising a memory unit (104) arranged to store information for several activity events, the apparatus (100) further comprising:

   a processing unit (102) that is coupled to the memory unit (104), wherein the processing unit (102) is configured to:

      (i) detect an activity event in acceleration measurements obtained by the accelerometer (108), wherein the subject is in one or more postures during the activity event;
      (ii) determine at least one quality indicator related to the detected activity event;
      (iii) determine whether each of the at least one quality indicators meets a respective predetermined criterion;
      (iv) responsive to at least one quality indicator meeting the respective predetermined criterion, decide whether information related to the detected activity event can be stored in the memory unit (104), and if so, store information related to the detected activity event in the memory unit (104) together with information related to one or more other activity events if any;
      (v) determine whether a calibration procedure should be performed based on information stored in the memory unit (104), and if so, process the stored information to determine a transformation matrix, wherein the transformation matrix relates a frame of reference of the accelerometer (108) to a frame of reference of the subject.

10. An apparatus (100) according to claim 9, wherein the processing unit (102) is further configured to:

      (vi) after operation (v), repeat operations (i)-(v) for additional acceleration measurements obtained by the accelerometer (108).

11. An apparatus (100) according to claim 10, wherein repeating operation (i) comprises:

      detecting an activity event by the subject using acceleration measurements obtained by the accelerometer (108) and the determined transformation matrix.

12. An apparatus (100) according to any of claims 9-11, wherein the at least one quality indicator is indicative of the suitability of the detected activity event for use in the calibration procedure.

13. An apparatus (100) according to any of claims 9-12, wherein the processing unit (102) is configured to determine whether information related to the detected activity event can be stored in a memory unit (104) by determining whether there is sufficient space in the memory unit (104) to store information related to the detected activity event; responsive to determining that there is sufficient space, storing information related to the detected activity event in the memory unit (104);

responsive to determining that there is insufficient space in the memory unit (104) for storing the information, comparing the at least one quality indicator related to the detected activity event to corresponding quality indicators related to a plurality of previously detected activity events for which information is stored in the memory unit (104); determining whether to store information related to the detected activity event in place of stored information for a previously detected activity event based on the comparison, and if so, storing the storing information related to the detected activity event in the memory unit (104), otherwise discarding information related to the detected activity event.

14. An apparatus (100) according to any of claims 9-13, wherein operation (v) comprises determining whether information related to at least a predetermined number of activity events has been stored in the memory unit (104).

15. An apparatus (100) according to any of claims 9-14, wherein the processing unit (102) is configured to determine whether the calibration procedure can be performed based on an amount of variation between respective mean gravity vectors for each activity event stored in the memory unit (104).

100

Fig. 1

202

204

206

208

210

Fig. 2

Fig. 3

Europäisches
Patentamt

European
Patent Office

Office européen
des brevets

# EUROPEAN SEARCH REPORT

Application Number

EP 18 20 6636

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/258374 A1 (LY DANIEL [US] ET AL) 14 September 2017 (2017-09-14) * throughout, e.g. [0012], [0015], [0016], [0027], [0042], [0052]-[0056], [0062], [0080]-[0086], [0095] *<br>----- | 1-15 | INV.<br>G16H40/63<br>G06F3/0346<br>A61B5/11 |
| X | US 2014/019080 A1 (CHAN ALEXANDER [US] ET AL) 16 January 2014 (2014-01-16) * throughout, e.g. [0004], [0020]-[0023], [0029]-[0031], claims 1-6 *<br>----- | 1-15 | |
| X | CN 105 606 846 A (BEIJING INST TECHNOLOGY) 25 May 2016 (2016-05-25) * throughout, e.g. abstract, claim 1 *<br>----- | 1-15 | |

TECHNICAL FIELDS
SEARCHED      (IPC)

G16H
B60G
G06F
A61B

The present search report has been drawn up for all claims

| Place of search | Date of completion of the search | Examiner |
|---|---|---|
| Munich | 14 May 2019 | Wimmer, Georg |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.........................................................................................
& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 18 20 6636

This annex lists the patent family members relating to the patent documents cited in the above-mentioned European search report.
The members are as contained in the European Patent Office EDP file on
The European Patent Office is in no way liable for these particulars which are merely given for the purpose of information.

14-05-2019

| Patent document cited in search report | | | Publication date | Patent family member(s) | | | Publication date |
|---|---|---|---|---|---|---|---|
| US 2017258374 | A1 | | 14-09-2017 | CN | 109310364 | A | 05-02-2019 |
| | | | | KR | 20190004697 | A | 14-01-2019 |
| | | | | SG | 11201807561Y | A | 30-10-2018 |
| | | | | US | 2017258374 | A1 | 14-09-2017 |
| | | | | WO | 2017156267 | A1 | 14-09-2017 |
| US 2014019080 | A1 | | 16-01-2014 | US | 9471541 | B1 | 18-10-2016 |
| | | | | US | 2014019080 | A1 | 16-01-2014 |
| | | | | US | 2017000410 | A1 | 05-01-2017 |
| | | | | US | 2017184630 | A1 | 29-06-2017 |
| | | | | WO | 2014011343 | A1 | 16-01-2014 |
| CN 105606846 | A | | 25-05-2016 | NONE | | | |

EPO FORM P0459

For more details about this annex : see Official Journal of the European Patent Office, No. 12/82

**REFERENCES CITED IN THE DESCRIPTION**

**Patent documents cited in the description**

- US 2013116602 A